# EUROPEAN PATENT APPLICATION

(11) **EP 1 832 310 A2**
(43) Date of publication of application: **12.09.2007**
(21) Application number: 07250909.4
(22) Date of filing: 06.03.2007
(51) Int. Cl.: A61N 1/05

(54) **Medical lead with tissue-protecting tip structure**

(30) Priority: 06.03.2006 US 369265
(71) Applicant: PACESETTER, INC., Sylmar, CA 91342-9221 (US)
(72) Inventor: Helland, John R., Saugus, California 91350 (US); Kistler, David E., Valencia, California 91355 (US)
(74) Representative: Rees, David Christopher

(57) **Abstract**

A transvenous cardiac lead (102) comprises a lead body (104) having a distal end portion (108) comprising a tip (130) adapted to be placed in engagement with cardiac tissue. A protective device (136) is carried by the distal end portion (108) of the lead body (104) in operative association with the tip (130), the protective device (136) being deployable following placement of the tip (130) to protect the cardiac tissue engaged by the tip (130). The deployable protective device (136) may be expandable upon implantation to provide a larger surface area or footprint about the lead tip (130) so as to retard or reduce the risk of penetration or perforation of the myocardium by the tip. The protective device (136) may comprise a matrix expandable in response to contact with bodily fluids.

## Description

The present invention relates in general to implantable medical devices, and more particularly to leads and catheters implantable in biological tissue such as the cardiac tissue of the heart. The invention is more specifically directed to a lead having at or adjacent to its tip an element manually or self-deployable to protect the cardiac tissue from being perforated or otherwise damaged by the tip after implant.

Various types of transvenous pacing and cardioversion/defibrillation leads have been developed for endocardial introduction into different chambers of a patient's heart, typically the right ventricle (RV) or right atrium (RA) appendage, as well as the coronary sinus (CS) veins. These flexible leads comprise lead bodies usually having an electrically insulating, outer polymer sheath or housing, such as a flexible silicone or polyurethane tube or coating, for encasing one or more electrical coil or cable conductors. In bipolar or multipolar leads, such electrical conductors are typically connected to tip, ring and cardioversion/defibrillator electrodes disposed along the lead body. The proximal ends of the conductors are coupled to an electrical connector at a proximal end of the lead body. The electrical connector may include a single pin contact in the case of unipolar leads, and additional pin or ring contacts in bipolar and multipolar leads.

The tip electrode is usually placed in contact with the heart's endocardial tissue by passage through venous access, often the subclavian or cephalic vein or one of its branches, which leads to the endocardial surface of the heart chambers. The tip electrode may be held in place passively, for example, by silicone or polyurethane tines within the trabeculae of the right ventricle (RV), in the apex, or in other appropriate sites. Alternatively, the tip electrode may be held in place actively through the use of a manipulated anchor or helical screw that penetrates the endocardial layer and into the myocardium. These fixation mechanisms help to prevent dislodgment of the tip, thus maintaining consistent sensing and pacing characteristics over time.

As cardiac leads have evolved over the years, an important goal has been to make such leads smaller in diameter. Small diameter leads are desirable because they take up less space in the venous system and can be implanted more easily in smaller veins. In addition, multiple, small diameter leads can be delivered through the same vessel without appreciably constricting blood flow.

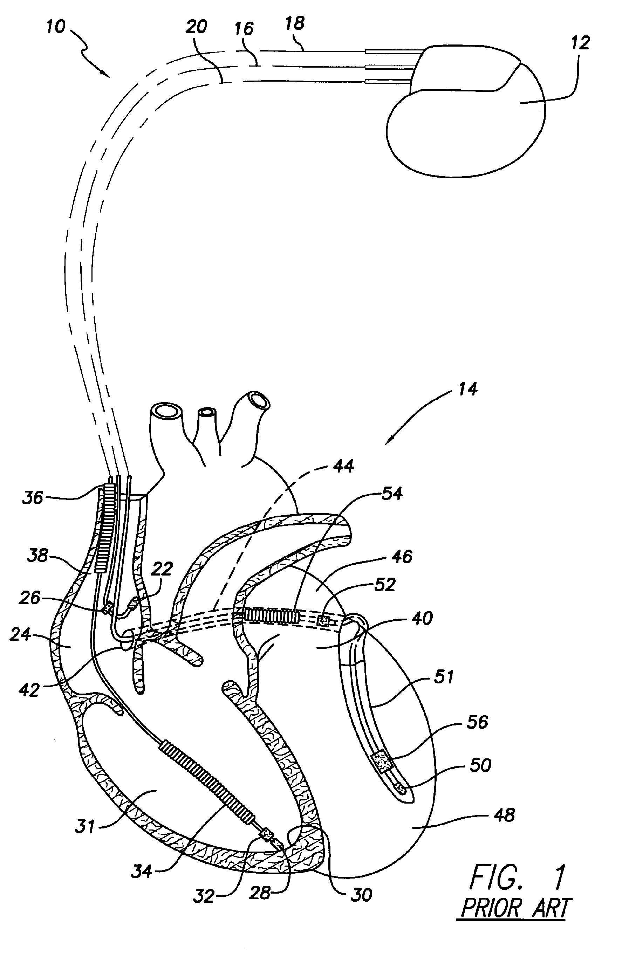

For example, **FIG. 1** shows a conventional system 10 for electrically stimulating the tissue of multiple cardiac chambers and for sensing electrical signals generated thereby. The system 10 comprises an implantable medical device 12 in electrical communication with a patient's heart 14 by way of three leads 16, 18 and 20, suitable for delivering multichamber stimulation, sensing and shock therapy. The lead 16, which senses right atrial cardiac signals and provides right atrial chamber stimulation therapy, comprises an atrial tip electrode 22, which typically is placed in contact with the tissue of the appendage of the RA 24 or at a site in the RA septal wall, and an atrial ring electrode 26. The lead 18 comprises a tip electrode 28 in engagement with the cardiac tissue at the apex 30 of the RV 31 or at an appropriate site in the RV outflow tract or septal wall, a ring sensing electrode 32 proximal of the tip electrode 28, and a shocking electrode 34 also within the right ventricle 31. The lead 18 may also carry a shocking electrode 36 in electrical communication with the superior vena cava (SVC) 38.

The lead 20 senses left atrial and ventricular cardiac signals and provides left chamber pacing therapy. The lead 20 is a CS lead designed for placement in the CS region 40 via the CS ostium 42 for positioning a distal electrode in the CS 44 adjacent to the left atrium 46 and/or additional electrode(s) adjacent to the LV 48. The exemplary CS lead 20 is designed to receive atrial and ventricular cardiac signals and to deliver left ventricular pacing therapy using at least a left ventricular tip electrode 50 placed, for example, in the left posterior ventricle vein 51.

It will be evident that since the three leads 16, 18 and 20 may all be passed to the heart through a single vessel, their diameters should be as small as possible. It will be equally evident that there will be a concomitant decrease in the size of the tip electrodes.

As lead body diameters have decreased to sizes currently in use, for example, generally in the range of 0.065-0.104 inch (5-8F), substantial increases in cardiac tissue perforations both acutely and chronically have been observed due to axial movements of the small, relatively sharp tip electrodes relative to the cardiac tissue engaged thereby akin to a "spiked heel" effect. Accordingly, as improved leads with downsized lead bodies continue to be developed, it is becoming increasingly important that such leads be designed to minimize the potential for acute and chronic perforations and penetrations of the myocardium.

According to the invention, there is provided a transvenous cardiac lead comprising a lead body having a distal end portion with a tip arranged to be placed in engagement with cardiac tissue; characterised by a protective device carried by the distal end portion of the lead body, the protective device being deployable following placement of the tip to protect the cardiac tissue engaged by the tip.

The protective device may protect against perforation of the cardiac tissue by the tip or penetration to an undesirable extent into the cardiac tissue. The protective device may be carried on the outer surface of the lead body, proximal of a tip electrode.

In accordance with one, specific, exemplary embodiment, there is provided a transvenous cardiac lead comprising a lead body having a distal end portion comprising a tip adapted to be placed in engagement with cardiac tissue. A protective device is carried by the distal end portion of the lead body in operative association with the tip, the protective device being deployable following placement of the tip to protect the cardiac tissue engaged by the tip.

In accordance with one illustrative embodiment, the protective device may comprise a matrix expandable in response to contact with bodily fluids. Preferably, the expandable matrix comprises hydrogel or other suitable biocompatible swellable soft material mounted on or adjacent to the distal tip of the lead. Such material, over a period of time such as, for example, 12-48 hours, swells and in effect expands to its natural *in vivo* state to form a larger footprint about the distal tip of the lead.

Pursuant to another illustrative embodiment, the protective device at or adjacent the tip of the lead may comprise at least one electroactive polymer element electrically connected to a proximal end of the lead body. In one form, the at least one electroactive polymer element may have an electrically inactive state wherein the element projects outwardly from an outer surface of the distal end portion of the lead body, and an electrically active state wherein the element lies adjacent to the outer surface so as to facilitate implantation of the lead transvenously. Upon placement of the lead, electrical activation is terminated and the electroactive polymer element deploys to its natural shape to form a larger footprint about the distal tip of the lead so as to provide the desired reduction or elimination of the potential for tissue penetration or perforation by the tip.

The invention may be carried into practice in various ways and some embodiments will now be described by way of example with reference to the accompanying drawings, in which:

**FIG. 1** is a diagrammatic, perspective view of the anterior portion of a human heart showing a conventional arrangement of multiple stimulation and sensing leads having tip electrodes in engagement with the tissue of various chambers of the heart;

**FIG. 2** is a side view of a bipolar lead system in accordance with one specific, exemplary embodiment of the invention;

**FIG. 3** is a side view of the distal end portion of a pacing and sensing lead forming part of the lead system of **FIG. 2**, the distal end portion including an expandable, cardiac tissue-protecting device proximal of the tip electrode of the lead;

**FIG. 4** is similar to the side view of **FIG. 3** showing the tip electrode in engagement with cardiac tissue and the tissue-protecting device in its deployed or expanded state;

**FIG. 5** shows an alternative form of the embodiment of **FIGS. 2-4**;

**FIG. 6** is a side view of the distal end portion of a lead comprising part of a bipolar lead system in accordance with another specific, exemplary embodiment of the invention including another form of an expandable, cardiac tissue-protecting device;

**FIG. 7** is similar to the side view of **FIG. 6** showing a tip electrode of the lead in engagement with cardiac tissue and the tissue-protecting device in its deployed or expanded state;

**FIG. 8** is a front view of the lead shown in **FIG. 7;**

**FIG. 9** is a side view of the distal end portion of a lead in accordance with yet another embodiment of the invention employing a helical, screw-in anchoring element carrying an expandable, cardiac tissue-protecting device; and

**FIG. 10** is a side view similar to that of **FIG. 9** showing the helical anchoring element screwed into cardiac tissue and the tissue-protecting device in its deployed or expanded state.

It will become evident from the following description that the invention has broad utility in that it may provide protection against the perforation of or other damage to body tissue in a wide variety of contexts, including but not limited to catheters with or without electrodes, and various lead types such as endocardial and epicardial leads. By way of example, the invention will be described principally as applied to transvenously delivered pacing and/or sensing leads implantable in the chambers of the right side of the heart or in the veins of the coronary sinus system.

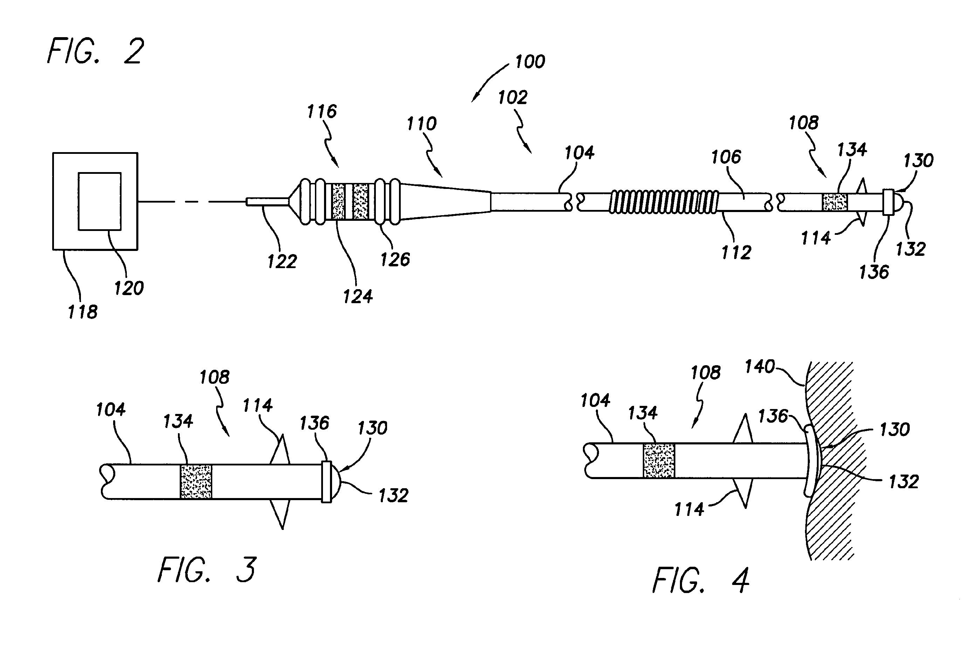

With reference to **FIGS. 2-4**, there is shown a medical lead system 100 comprising a bipolar endocardial pacing and sensing lead 102 in accordance with one specific, exemplary embodiment of the present invention. The lead 102 includes a lead body 104 comprising an outer surface 106, a distal end portion 108 and a proximal end portion 110. The lead body 104 may comprise a tubular, insulating sheath or housing 112 made of a biocompatible, biostable, polymer such as silicone rubber or polyurethane or hybrids thereof.

In accordance with current medical lead technology, the lead body 104 may have a small outside diameter ranging, for example, from about 0.026 inch (2.0 F) to about 0.091 inch (7.0 F).

The distal end portion 108 of the lead body may include passive fixation means to stabilize the distal end portion of the lead relative to the cardiac tissue. For example, the passive fixation or stabilizing means may comprise one or more soft, flexible protuberances or tines 114 on the distal end portion of the lead body.

Attached to the proximal end portion 110 of the lead body 104 is an electrical connector assembly 116 adapted to be received by a receptacle in a permanent or temporary medical device 118 containing appropriate pacing, sensing, shocking and other electrical circuitry 120 all in accordance with techniques and principles well-known in the art.

The connector assembly 116 includes a tubular terminal contact pin 122 and a terminal contact ring 124. The connector assembly is received within a receptacle of the device 118. To prevent ingress of body fluids into the receptacle, the connector assembly 116 may be provided with spaced sets of seals 126 in accordance with well-known arrangements in the art. Further, in accordance with well-known implantation techniques, a stylet or guide wire (not shown) for delivering and steering the distal end portion 108 of the lead body during implantation may be inserted into the lead body through the tubular connector pin 122 and through a central lumen extending longitudinally along the length of the lead body. Preferably, the lead body provides the option of using either an over-the-wire (OTW) or stylet driver approach to lead placement.

The distal end portion 108 of the lead body 104 has a distal extremity or tip 130 that may incorporate a cathodal tip electrode 132. For bipolar pacing and sensing, an anodal ring electrode 134 may be disposed proximally of the tip electrode 132 and spaced apart therefrom. As is well known, the tip electrode 132 may be made of a platinum-iridium alloy or similar biostable, biocompatible, low polarization, conductive material. The tip electrode has an active exterior surface that may be treated and/or configured in well-known ways for increasing stimulation efficiency, reducing electrode polarization, providing automatic capture compatibility, and/or presenting a roughened surface adapted to promote tissue in-growth to help prevent dislodgement of the tip electrode.

Given the small diameter of the lead body, the area of the stimulating or active tip electrode surface may range from about 1.0 mm² to about 10 mm² and more preferably in the range of 2.5 to 5 mm². As noted, it is this small area or footprint that gives rise to the potential problem of cardiac tissue penetration addressed by the present invention.

In accordance with the embodiment shown in **FIGS. 2-4**, mounted on the outer surface of the distal end portion 108 of the lead body at or adjacent and proximal to the tip electrode 132 is an expandable, tissue-protecting device 136 in the form of a matrix comprising a hydrogel or other swellable, biocompatible substance in the form of an annular ring or collar. As used herein, the term "hydrogel" refers to a superabsorbent material comprising a structure or matrix, typically a natural or synthetic polymer, that expands in volume upon hydration from body fluids.

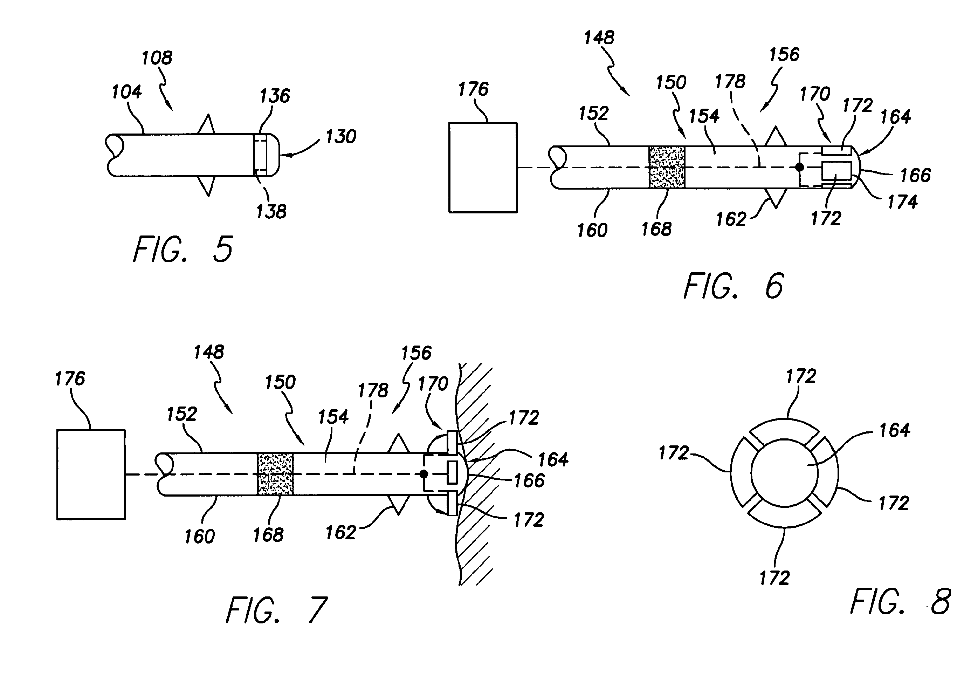

Before implantation (**FIGS. 2** and **3**), the hydrogel layer 136 comprises a relatively thin annular coating or ring on the outer surface of the distal end portion of the lead body immediately adjacent to the tip electrode. Alternatively, the unexpanded hydrogel layer may be contained within a shallow, annular groove or recess 138 formed in the outer surface of the lead body so that the hydrogel layer and outer lead body surface 106 are substantially flush. (**FIG. 5**.) After implantation, the hydrogel layer absorbs body fluids and expands in volume about the tip of the lead (**FIG**. **4**). In the hydrogel layers of the present invention, hydrogels having a thickness increase greater than about 50 percent are preferred.

As seen in **FIG. 4**, the primary function of the protective hydrogel device 136 in accordance with the present invention is to provide an enlarged area cushion or pillow that helps to protect the cardiac tissue 140 from being penetrated by the relatively sharp tip of the lead.

The following are some examples of hydrogels that may be used for the expandable protective device of the invention: poly 1-hydroxyethyl methacrylate, polymethacrylic acid, poly(N,N, dimethylaminoethyl methacrylate), polyacrylamide, poly(N-vinyl pyrrolidone), polyvinyl alcohol, polyethylene oxides, hydrolyzed polyacrylonitrile, polyelectrolyte complexes, polymethacrylic acid and polyacrylonitrile, anionic and cationic hydrogels or composites or copolymers of one or more of these hydrogels together with other suitable biocompatible materials such as silicone or polyurethane.

In one embodiment, the hydrogel protective device 136 may also be used as a vehicle for drug delivery by loading a polymeric hydrogel precursor with a blood soluble or insoluble drug, or by chemically binding a drug such as an antibiotic, an antiseptic, an anti-arrhythmic, anti-inflammatory steroid or other agent to the polymer network. Upon expansion of the hydrogel layer, the drug is also released to the adjacent tissue.

In appropriate instances, such as when the hydrogel is intended to swell up to mask off a portion of the electrode's active surface, the hydrogel protective device 136 may also be loaded with an additive, such as an electrolyte, to enhance its electrical conductivity.

Other materials may be incorporated into the expandable matrix to yield additional advantages or results. For example, in one embodiment, the expandable matrix may incorporate a radiopaque material such as barium sulfate so that the device 136 may be fluoroscopically visualized.

The hydrogel protective device 136 may also be blended with a non-hydrogel polymer that increases the physical strength of the device and/or helps control the degree of swelling. Methods for preparing hydrogel/non-hydrogel polymer blends are well known in the art.

The biocompatibility and dimensional and chemical stability of hydrogels render them well suited for long term implantation. The expanded hydrogel device 136 may also inhibit tissue adhesion and thrombus formation on the lead tip.

The hydrogel may also be blended with an echographic substance to allow the matrix to be detected and visualized using echocardiography.

Still further, fibrin or other biocompatible glue may be carried by the hydrogel to toughen or strengthen the tissue adjacent to the lead tip.

In one embodiment, the hydrogel protective layer may be initially formed on the lead body adjacent to the tip electrode by dip-coating unmasked areas or predetermined parts of the lead body surface in a hydrogel solution. Other exemplary coating techniques include molding, spraying and vapor deposition, and mechanical means such as press-fitting the matrix to the lead body, or bonding it to the lead body. Still further coating and application techniques will suggest themselves to those skilled in the art. Further, it may be desirable to enhance the adhesion of the hydrogel protective device to the outer surface of the lead body. Several methods are known in the art for accomplishing such a result including, for example, ion etching of the surface, thermal or chemical surface treatment, radiation, and applying a primer layer coating to the outer surface of the lead body.

Preferably, the dry, unexpanded hydrogel layer may have a thickness in the range of 0.005 to 0.020 inch and the hydrated expanded thickness may be approximately 0.025 to 0.060 inch, and more preferably about 0.030 to 0.050 inch for a lead having an outer diameter of 5 F, by way of example. Accordingly, a typical dry, hydrogel coated distal end portion of the lead body may have an initial outer diameter of approximately the diameter of the lead, or slightly larger. When the hydrogel protective device is expanded, the final outer diameter thereof may be about 30% or more, greater than the diameter of the lead. When the hydrogel protective device becomes hydrated, the increase in the hydrogel layer thickness is preferably greater than 30 percent.

With reference to **FIGS. 6-8**, there is shown a medical lead system 148 comprising a bipolar endocardial pacing and sensing lead 150 in accordance with another exemplary embodiment of the present invention. The lead 150 includes a lead body 152 comprising an outer surface 154, a distal end portion 156 and a proximal end portion (not shown) carrying an electrical connector assembly for coupling the lead body to a medical device such as a pulse generator. The lead body may comprise a tubular, insulating sheath or housing 160 made of a biocompatible, biostable polymer such as silicone rubber or polyurethane.

As before, the distal end portion 156 of the lead body may include passive fixation means in the form of soft, flexible protuberances or tines 162 that aid in stabilizing the position of the distal end portion of the lead body relative to target cardiac tissue whose electrical activity is to be stimulated and/or sensed by the lead.

As before, the distal end portion 156 of the lead body 152 has a distal extremity or tip 164 that may incorporate a cathodal tip electrode 166. For bipolar pacing and sensing, an anodal ring electrode 168 may be disposed proximally of the tip electrode 166 and spaced apart therefrom.

For the small diameter leads that are currently in use within the cardiac rhythm management industry, the area of the stimulating or active tip electrode surface may be in the range of 1.0 mm² to about 10 mm². Moreover, the distal tip of the lead, whether comprising an electrode or not, may often be only 1 to 4 mm² in cross-sectional area. As already indicated, it is this small surface area that raises the risk of perforation or penetration of the cardiac tissue by the tip electrode.

Accordingly, the distal end portion of the lead body includes on the outer surface thereof a deployable or expandable cardiac tissue-protecting device 170 comprising at least one electroactive polymer element, and preferably a plurality of electroactive polymer elements 172 each attached to the outer surface of the distal end portion of the lead body along a distal edge 174 of the element adjacent to the tip electrode. The electroactive polymer elements 172 are electrically coupled, preferably in parallel, to an electrical power supply 176 by conductors 178 for electrical activation of the elements. Such activation may be provided by, for example, a clip attachable to the pin contact on the connector assembly at the proximal end of the lead body. In the default or electrically inactive state of the electroactive polymer elements, the elements are bent outwardly to assume a deployed or expanded state as shown in **FIGS. 7** and **8**. Upon implantation, the elements 172 are electrically activated as described so that they flatten and lie against the outer surface of the distal end portion of the lead body **(FIG. 6**) or within shallow recesses (not shown) formed therein so that the outer surfaces of the elements are flush or substantially flush with the outer surface of the lead body along the lines described in connection with **FIG. 5.** Removal of the electrical power supply causes the electroactive polymer elements to assume their electrically inactive or default state, that is, to deploy thereby forming an enlarged, generally radially extending surface area adjacent and immediately proximal to the tip electrode thereby resisting axial movement of the distal end portion of the lead body relative to the cardiac tissue, which movement might otherwise cause the tip electrode to perforate or penetrate the cardiac tissue.

Electroactive polymers, also referred to as electrically conductive or conducting polymers, are flexible materials capable of converting energy in the form of electric charge and voltage to mechanical force and movement. Thus, these materials are able to change shape in response to electrical stimulation. Electroactive polymer materials are well known and for purposes of the present invention, may comprise, without limitation, polyaniline, polypyrrole, polysulfone and polyacetylene. It is well known that dimensional changes may be effected in these polymers by the mass transfer of ions into or out of the polymer that causes expansion or contraction thereof.

lonomeric polymer-metal composites (IPMC) comprise a sub-classification of ionic electroactive polymers. A typical IPMC consists of a thin polymer membrane with metal electrodes plated on both faces. The polyelectrolyte is neutralized with counter-ions, balancing the charge of the anions covalently fixed to the membrane. When an IPMC is hydrated and stimulated by a small voltage, for example, 1-5 volts, both the fixed anions and the mobile counter-ions are subjected to the electric field. The counter-ions diffuse toward one of the electrodes and, as a result, the composite undergoes a fast bending deformation toward the anode. The bending (in this case, a flattening of a pre-bent electroactive polymer element) is the result of increased stiffness along the cathode and decreased stiffness along the anode.

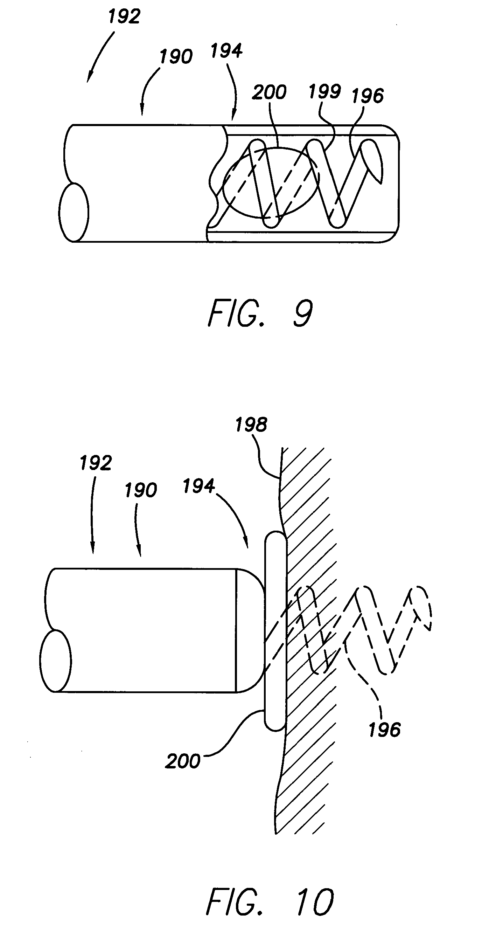

With reference to **FIGS. 9** and **10**, there is shown the distal end portion 190 of a unipolar or bipolar pacing/sensing or implantable cardioverter/defibrillator (ICD) lead body 192 having a tip 194 comprising an extendable, helical, pointed screw or helix 196 rotatable either by rotating the lead's connector assembly pin contact and associated conductor (which are attached to the helix), or with the aid of a stylet, so as to affix the lead body relative to the cardiac tissue 198 to be stimulated and/or sensed. As is well-known, the helical screw-in element 196 may be electrically inactive, thereby serving solely as an anchoring or fixation element or it may be electrically active in which case the helical screw-in element also serves as a tip electrode.

In accordance with the present invention, a cardiac tissue-protecting expandable matrix device 200 of hydrogel or a similar expandable matrix, as described above, is carried on or within the helical screw-in element 196, preferably within the lumen thereof. As shown in **FIG. 10**, with the helical fixation screw-in element 196 extended and screwed into the cardiac tissue 198, the hydrogel or expandable matrix engages the cardiac tissue and simultaneously begins deployment or expansion as it absorbs bodily fluid inside the chamber of the heart that receives the lead body. The increased area of the matrix device forms a cushion or pillow tending to resist axial movement of the lead body tip 194 relative to the cardiac tissue so as to protect the tissue from injury.

The expandable matrix device is preferably disposed at a predetermined position along the length of the helical screw-in element 196 at the time of the lead's manufacture. (**FIG. 9**.) Upon implantation, the helical screw-in element 196 is extended from the lead body (**FIG. 10**) carrying with it the expandable device which is correspondingly moved out of the lead body with the helical element 196 at a longitudinal position within or on the element 196 so as to allow the element 196 to become embedded to its intended full depth.

## Claims

1. A transvenous cardiac lead (102) comprising a lead body (104) having a distal end portion (108) with a tip (130) arranged to be placed in engagement with cardiac tissue; **characterised by** a protective device (136) carried by the distal end portion (108) of the lead body (104), the protective device (136) being deployable following placement of the tip (130) to protect the cardiac tissue engaged by the tip (130).

2. A lead as claimed in Claim 1, **characterised in that** the protective device (136) is radially expandable to assume an expanded state following placement of the tip (130).

3. A lead as claimed in Claim 1 or Claim 2, **characterised in that** the protective device (136) comprises a matrix expandable in response to contact with bodily fluids.

4. A lead as claimed in Claim 3, **characterised in that** the expandable matrix expands at least 30% when contacted by bodily fluids.

5. A lead as claimed in Claim 3 or Claim 4, **characterised in that** the expandable matrix carries a material selected from the group consisting of an electrolyte, a drug, a radiopaque substance and an echographic substance.

6. A lead as claimed in any of Claims 2 to 5, **characterised in that** the expandable matrix comprises hydrogel.

7. A lead as claimed in Claim 6, **characterised in that** the hydrogel is blended with a non-hydrogel polymer.

8. A lead as claimed in Claim 1 or Claim 2, **characterised in that** the protective device (136) comprises at least one electroactive polymer element (172) electrically connected to a proximal end (110) of the lead body (104).

9. A lead as claimed in Claim 7, **characterised in that** the at least one electroactive polymer element (172) has a normally deployed state in the absence of an electrical signal and a non-deployed state in response to the application of an electrical signal.

10. A lead as claimed in any preceding claim, **characterised in that** the tip (130) comprises a tip electrode (132) and/or a helical screw-in element (196).

11. A lead as claimed in Claim 10, **characterised in that** the protective device (200) is carried by the helical screw-in element (196).

12. A lead as claimed in any preceding claim, **characterised in that** the lead body (104) has an outer surface (106), a distal end portion (108), and a proximal end (110) carrying at least one electrical contact connected to a tip electrode (132) adapted to be placed in engagement with cardiac tissue upon implantation; and the protective device (136) is carried by the outer surface (106) of the lead body (104) proximal of the tip electrode (132), the protective device (136) being deployable after implantation to protect the cardiac tissue engaged by the tip electrode (132).

13. A lead as claimed in Claim 12, **characterised in that** the at least one electroactive polymer element (172) is attached to the outer surface (106) of the lead body (104) along a distal edge of the element.
